(19) Europäisches Patentamt
European Patent Office
Office européen des brevets

(11) Veröffentlichungsnummer: **0 253 199**
**A2**

(12) # EUROPÄISCHE PATENTANMELDUNG

(21) Anmeldenummer: 87109377.9

(22) Anmeldetag: 30.06.87

(51) Int. Cl.4: **C12P 1/00**, C12M 1/40, C12P 13/04, C12P 41/00

(30) Priorität: **05.07.86 DE 3622662**

(43) Veröffentlichungstag der Anmeldung: **20.01.88 Patentblatt 88/03**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Müller, Wolfgang, Dr.**
**Theodor-Körner Strasse 12**
**D-6238 Hofheim an Taunus(DE)**
Erfinder: **Keller, Reinhold, Dr.**
**Wiesenweg 5**
**D-6232 Bad Soden am Taunus(DE)**
Erfinder: **Wullbrandt, Dieter, Dr.**
**Bienerstrasse 29**
**D-6238 Hofheim am Taunus(DE)**

(54) **Verfahren zur kontinuierlichen biokatalytischen Umsetzung von Substraten, die in wässrigen Lösungen schwer löslich sind.**

(57) Die Erfindung stellt ein Verfahren zur kontinuierlichen biokatalytischen Umsetzung von Substraten, die in Wasser schwerlöslich sind, in wäßriger Lösung dar. Das in einem Behälter befindliche Substrat wird kontinuierlich mittels einer wäßrigen Lösung herausgelöst und über einen nachgeschalteten Behälter gegeben, der mit einem Biokatalysator beschickt ist.

**EP 0 253 199 A2**

# Verfahren zur kontinuierlichen biokatalytischen Umsetzung von Substraten, die in wäßrigen Lösungen schwer löslich sind.

Bisher sind keine kontinuierlichen Verfahren zur biokatalytischen Umsetzung von schwer wasserlöslichen Substraten in wäßriger Lösung bekannt. In der U.S. Patentschrift 4.262.092 wird die Spaltung von D,L-N-Acetylphenylalaninmethylester mit Alcalase im rein wäßrigen System beschrieben. Die Trennung der Spaltprodukte erfolgt durch Extraktion mit organischen Lösemitteln. Das Verfahren wird diskontinuierlich ohne Wiederverwendung des Enzyms durchgeführt, wodurch der Prozeß unwirtschaftlich wird. Die Spaltung von D,L-N-Acetylphenylglycinalkylestern wird nach der Deutschen Patentanmeldung 2 927 535 mit an DEAE-Sepharose immobilisiertem Subtilisin im 2 Phasensymstem erreicht, das aus Wasser und einem organischen Lösungsmittel besteht. Das organische Lösungsmittel wird eingesetzt, um die Löslichkeit des Substrats zu erhöhen. Dies hat aber gleichzeitig den Nachteil, daß die Aktivität des Enzyms signifikant erniedrigt wird.

Zur Umgehung der beschriebenen Nachteile der bekannten Prozesse wurde ein Verfahren entwickelt, mit dessen Hilfe Substrate, die in Wasser schwerlöslich sind, ohne Zusatz von organischen Lösemitteln kontinuierlich in wäßrigen Lösungen mit Biokatalysatoren umgesetzt werden können.

Die Erfindung betrifft somit ein Verfahren zur kontinuierlichen biokatalytischen Umsetzung von in Wasser schwerlöslichen Substraten, das dadurch gekennzeichnet ist, daß eine wäßrige Lösung kontinuierlich das in einem Behälter befindliche Substrat herauslöst und über den in einem nachgeschalteten Behälter befindlichen Biokatalysator führt. Die Erfindung betrifft ferner die Vorrichtung, mit deren Hilfe das Verfahren durchgeführt werden kann.

Im folgenden werden bevorzugte Ausführungen der Erfindung detailliert beschrieben. Ferner wird die Erfindung in den Ansprüchen definiert.

Das in der Abbildung dargestellte Fließschema zeigt die Anordnung der Vorrichtung. Die Substratvorratsgefäße (1a, b usw.) können in beliebiger Zahl hintereinander geschaltet sein. Die Gefäße bestehen aus inerten Materialien, die das Substrat in keiner Weise verändern. Bevorzugt wird Glas verwendet. Die Gefäße sind vorzugsweise säulenförmig und über ihre ganze Länge mit einem Kühl/Heizmantel umgeben. Im unteren Teil der Gefäße befindet sich ein Metallsieb oder eine Fritte (2a, b), deren Durchlässigkeit so gewählt wird, daß ein Abfließen des festen Materials aus dem Vorratsgefäß verhindert wird. Feste Substrate können direkt in die Gefäße eingefüllt werden. Ölige Substrate werden auf eine grobe inerte Matrix, beispielsweise Kieselgelgranulat mit einem Durchmesser von 1 bis 5 mm, bevorzugt 2 bis 4 mm, aufgezogen und dann in den Behälter gegeben. Räumlich getrennt von dem Substratvorratsgefäß befindet sich ein weiteres Gefäß (3), das mit dem Biokatalysator beschickt ist. Das Gefäß besteht ebenfalls aus inertem Material, bevorzugt Glas, mit einem dem Auslauf vorgeschalteten Sieb (4), das ein Auswaschen des Säuleninhalts verhindert. Das Gefäß ist bevorzugt säulenförmig und mit einem Kühl/Heizmantel umgeben. In dem nachgeschalteten Titrationsgefäß (5), das mit einem Rührer (6) versehen, ist erfolgt die Nachjustierung des pH-Wertes (7), sowie mit Hilfe einer Pumpe (8) die Produktentnahme (9) und mit einer weiteren Pumpe (10) die Zufuhr (11) wäßriger Lösung. Das Titrationsgefäß besteht aus inertem Material und ist ebenfalls mit einem Kühl/Heizmantel umgeben. Die Umwälzpumpe (12) pumpt die wäßrige Lösung im Kreislauf durch eine inerte Schlauchleitung (13) vom Titrationsgefäß über das Substratvorratsgefäß, das über einen Dreiwegehahn (14a) mit dessen Zulauf (15a, b usw.) verbunden ist. Von dem Substratgefäß wird weiter über einen Ablauf (16a, b usw.) sowie über eine inerte Schlauchleitung (17), die mit dem Ablauf über einen Dreiwegehahn (14b) verbunden ist, durch die Biokatalysatorsäule über die inerte Schlauchleitung (18) in das Titrationsgefäß gepumpt.

Zunächst wird lediglich das Substratvorratsgefäß 1a in den Kreislauf eingeschaltet. Wasser bzw. die wäßrige Lösung, in der die Substratumsetzung stattfinden soll, wird über das Substrat gegeben, wobei man nach kurzer Zeit durch die Extraktion des festen Substrats eine gesättigte Lösung erhält. Diese wird dann über die Biokatalysatorsäule gegeben, in der die Umsetzung des Substrats stattfindet. Der kontinuierliche Extraktionsprozeß sorgt für ein stetiges Lösen des Substrats, so daß immer Material im Kreislauf zirkuliert, das umgesetzt werden kann. Steht kein Substrat aus 1a mehr zur Verfügung, wird Ib dazugeschaltet usw.

Dieses Verfahren ist grundsätzlich auf alle biokatalytischen Umsetzungen von Substraten anzuwenden, die in Wasser nur schwer löslich sind. Unter Biokatalysatoren sind beispielsweise Enzyme oder Mikroorganismen, die vorzugsweise immbilisiert sind, zu verstehen und mit denen entsprechende Reaktionen durchgeführt werden können. Die für die Reaktion optimalen Bedingungen sind dem Fachmann entweder bekannt oder können in kurzen Vorversuchen ermittelt werden.

Besonders vorteilhaft ist das Verfahren für die Umsetzung optisch aktiver Verbindungen bzw. die Racematspaltung einzusetzen, insbesondere für die Spaltung aromatischer D,L-N-Acylaminosäureester der allgemeinen Formel I,

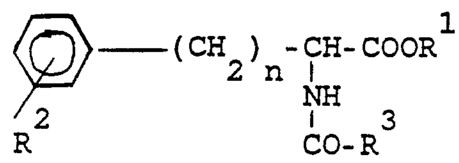

I

in der bevorzugt $R^1$ ($C_1$-$C_5$)Alkyl, $R^2$ Wasserstoff oder Hydroxyl, $R^3$ ($C_1$-$C_5$)Alkyl, Benzyl oder ($C_1$-$C_5$)-Alkoxy bzw. Benzyloxy und n 0, 1 oder 2 bedeutet. Ebenfalls vorteilhaft kann mit dem erfindungsgemäßen Verfahren die Monoesterspaltung der L-Form acylierter $\alpha$-Amino-dicarbonsäuren der allgemeinen Formel II,

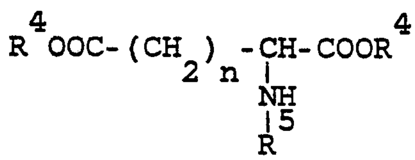

II

in der bevorzugt $R^4$ ($C_1$-$C_6$)-Alkyl, $R^5$ Wasserstoff oder eine der Acylgruppen $COCH_3$, $COCH_2Cl$, $COC_2H_5$ oder $COC_2H_4Cl$ oder eine Gruppe vom Urethantyp und n 1 oder 2 bedeutet, durchgeführt werden.

Mittels einer Esterase, beispielsweise Serinproteasen wie $\alpha$-Chymotrypsin, bevorzugt eines an einen anorganischen Träger, gemäß der Europäischen Patentanmeldung 3 438 189, immobilisierten $\alpha$-Chymotrypsin, erhält man durch enantioselektive Esterspaltung aus der L-Form der Verbindung der allgemeinen Formel I die optisch reinen L-Aminosäurederivate der allgemeinen FormelIII,

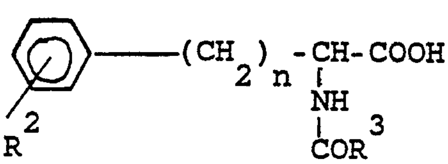

III

mit der obengenannten Bedeutung für $R^2$, $R^3$ und n, die durch Folgereaktionen in die entsprechenden freien L-Aminosäuren überführt werden können. Es können auch die entsprechenden Verbindungen eingesetzt werden, in denen die Aminogruppe ungeschützt ist. Besonders bevorzugte Substrate sind vor allem Tryptophan, N-Acetyl-phenyl-glycinethylester, Phenylalanin, Tyrosin, 3,4-Dihydroxy-Phenylalanin und Homophenylalanin mit geschützter und ungeschützter Aminogruppe.

Durch die oben beschriebene Extraktion von festem Racemat der Formel I mit wäßriger Lösung erhält man eine gesättigte Lösung dieser Verbindung, die über die mit Enzym beschickte Säule gegeben wird. Die Umsetzung erfolgt zweckmäßig bei 20° bis 40°C, vorteilhaft bei 30°C, und einem pH-Wert im Bereich von 5 bis 8. Durch die enzymatische Spaltung des Racemats verarmt die Lösung an L-Enantiomer, da dieses zur entsprechenden Säure gespalten wird. Da das Extraktionssystem stets im Sättigungsbereich des Racemats arbeitet, scheidet sich der überwiegende Anteil des nicht umsetzbaren D-Enantiomers durch Kristallisation ab. Die Kristallisation erfolgt bevorzugt dort, wo die lokale Konzentration des Racemats besonders hoch ist bzw. wo Kristallisationskeime vorhanden sind, z.B. bevorzugt im Substratvorratsgefäß. In Abhängigkeit vom Löslichkeitsprodukt verbleibt stets ein Restgehalt an gelöstem D-Ester im System, der sich ebenso wie die L-Säure in der wäßrigen Lösung befindet. Die kontinuierlich abgepumpte Lösung wird bei pH 7 mit geeigneten Lösemitteln, beispielsweise Methylenchlorid oder Ethylacetat, von restlichem Ester befreit. Die verbleibende wäßrige Phase wird auf pH 2 eingestellt und wiederum extrahiert. Diese Extrakte enthalten die L-Säure, die wiederum im Falle der N-Acylaminosäure, z.B. durch Säurehydrolyse, in die reine L-Aminosäure überführbar ist. Der D-Ester kann nach der Racemisierung, die bevorzugt gemäß der Europäischen Patentanmeldung 3 438 189 durchgeführt wird, wieder in den Kreislauf zurück geführt werden.

Ebenso erhält man durch selektive Esterspaltung der L-Form der Verbindung der allgemeinen Formel II die entsprechenden Monoester der allgemeinen Formel IV,

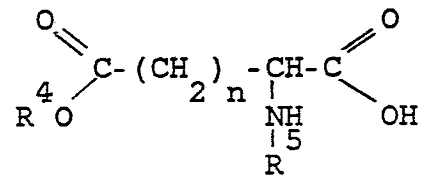

IV

in der $R^4$, $R^5$ und n die obengenannte Bedeutung haben. Die Hydrolyse der Aminosäurediester der allgemeinen Formel II wird mit Serinproteasen, bevorzugt mit immobilisiertem $\alpha$-Chymotrypsin, erfindungsgemäß durchgeführt. Es können sowohl das reine L-Enantiomer als auch das D,L-Racemat der Aminosäurediester bzw. ihrer N-Acylderivate eingesetzt werden, wobei jedoch nur die L-Form hydrolysiert wird. Das Verfahren eignet sich daher auch zur Racematspaltung. Die Reaktion läuft bei pH-Werten von 5 bis 8 und bei Temperaturen von 20 bis 40°C ab.

Das erfindungsgemäße Verfahren erlaubt die enzymatische Umsetzung unter für das Enzym günstigsten Bedingung, da in wäßriger Lösung gearbeitet werden kann. Insbesondere bei immobilisierten Enzymen bedeutet das im Vergleich zu Verfahren, in denen die Biokatalysatoren mit organischen Lösemitteln in Berührung gebracht werden, eine Erhöhung der Standzeiten und der Raum/Zeit-Ausbeuten.

Anhand der folgenden Beispiele soll die Erfindung in Einzelheiten dargestellt werden.

Beispiel 1

Racematspaltung von D,L-N-Acetyl-phenylalaninmethylester

Aufbau der Vorrichtung:

Als Substratgefäße werden mit einem Heizmantel umgebene Glassäulen mit einer Höhe von 30 cm und einem Durchmesser von 4 cm, das einem Gefäßvolumen von 400 ml entspricht, verwendet. Dem Ablauf der Gefäße ist eine G2 Fritte vorgeschaltet. Als Biokatalysatorsäule dient ebenfalls eine mit einer G2-Fritte versehene heizbare Glassäule mit einer Höhe von 9 cm und einem Durchmesser von 3 cm. Das Titrationsgefäß mit einem Fassungsvermögen von 400 ml besteht ebenfalls aus Glas. Ein diesem Gefäß zugeordneter Autotitrator (Metrom 655 Dosimat, Herisau/Schweiz) titriert die aus der Enzymsäule zufließende wäßrige Lösung mit 5N NaOH auf einen Sollwert von pH 7. Die genannten Gefäße werden mit PVC Schläuchen (Durchmesser 8 mm) über eine Membranpumpe (Typ: R 409 W, Fa. Immenhausen, Kassel) miteinander verbunden. Es wird mit einer Umpumpgeschwindigkeit von 3,7 Liter/h gearbeitet. Dem Titrationsgefäß sind weiterhin 2 mit einer Schlauchpumpe (LKB 2115, Bromma/Schweden) versehenen Silicon Leitungen (Durchmesser 1,3 mm) zugeordnet über die das Endprodukt abgepumpt werden kann, bzw. frische wäßrige Lösung kontinuierlich zugeführt wird. Die Zu-bzw. Ablaufgeschwindigkeit beträgt 2,5 ml/min.

Die Analytik der Endprodukte aus dem Titrationsgefäß erfolgt durch HPLC auf einer RP-18 Säule [Fließmittel: Methanol (2,5 l), $H_2O$ (2,5 l), 12,5 g $KH_2PO_4$, 2,5 g Tetrabutylammoniumhydrogensulfat, mit 40 % $H_3PO_4$ auf pH 2,82 eingestellt] mit Hilfe eines UV-Detektors bei 210µ.

Betriebsweise:

Für diesen Versuch werden insgesamt 4 Substratpatronen (1a, b, c, d) hintereinander geschaltet.

Die Substratvorratsgefäße werden wie folgt gefüllt:

1a mit 44,26 g (0,2 mol), 1b mit 66,39 g (0,3 mol), 1c und 1d mit 88,52 g(0,4 mol)

D,L-N-Acetylphenylalaninmethylester. Das Substrat wird zuvor mit 200 bis 400 ml bidestilliertem Wasser zu einem gießfähigen Brei verarbeitet und mit wenig 0,1 M K-Phosphatpuffer (pH 7) auf einen pH-Wert von 7 eingestellt. Man schaltet das Substratgefäß 1a, das auf eine Temperatur von 30°C gebracht wird, in den Kreislauf ein und pumpt von einem im Titrationsgefäß befindlichen Vorrat soviel Wasser zu, bis das System

vollständig gefüllt ist (Gesamtmenge $H_2O$: ca. 500 ml). Es wird 15 Minuten im Kreislauf gepumpt bis sich im System eine Substrat/Sättigungskonzentration von 2,2 % eingestellt hat. Dann wird die Biokatalysatorsäule, die mit 25 ml immobilisiertem α-Chymotrypsin beschickt ist, das gemäß der Europäischen Patentanmeldung 3 438 189, Beispiel 1, hergestellt wurde, in den Kreislauf eingeschaltet. Die Reaktion in der Enzymsäule findet bei 30°C statt. Die Prozeßsteuerung wie Umpump-, Zulauf-und Ablaufgeschwindigkeit erfolgt manuell. Die HPLC Analytik liefert Daten über Konzentrationen von Ester bzw. Säure.

Das Titrationsgefäß wird auf einer Temperatur von 30°C gehalten. Zu Beginn der Reaktion ist das Konzentrationsverhältnis von Ester : Säure ungefähr 1 : 1. Die enzymatische Spaltung des L-Esters verläuft sehr rasch und mit hohem Umsatz. Dies bestätigt die Überprüfung der optischen Reinheit des im Kreislauf zirkulierenden Esters, der zu 93,7% aus D-Ester besteht. Mit fortlaufender Reaktionszeit erreicht das Konzentrationsverhältnis einen Wert von 20 : 80. Man kann eine deutliche Kristallabscheidung des D-Esters im Substratgefäß beobachten. Zu diesem Zeitpunkt wird aus dem Titrationsgefäß Reaktionslösung kontinuierlich abgezapft bzw. mit der gleichen Geschwindigkeit Wässer hinzugepumpt.

Folgende Werte wurden anhand der ablaufenden Reaktionslösung ermittelt:

Konzentration des D-Esters: 6,2 - 7,4 g/L

" der L-Säure: 31,2 g/L

Spaltgeschw.: $\frac{0{,}22 \text{ mol D-Ester}}{\text{h}}$

Raum/Zeit-Ausbeute: $\frac{1{,}4 \text{ kg Phe}}{\text{h x Liter}}$

Der enzymatische Spaltprozeß kommt zum Erliegen, wenn kein spaltbarer L-Ester mehr gelöst vorliegt. In der Substratpatrone 1a befindet sich 15,45 g nicht-spaltbarer D-N-Acetyl-phenylalaninmethylester: $\alpha_D^{26}$ = -13,6° (c 0,5 in Ethanol)

Reinheit: (HPLC: nach "pre column" Derivatisierung mit o-Phthaldialdehyd / BOC-L-Cystein): 99,1 % D-Form.

Verarmt das System an spaltbarem L-Ester, so nähert sich die Titrationskurve asympthotisch einem Grenzwert. Sinnvollerweise vermeidet man dies, durch rechtzeitiges Zuschalten der neuen Substratpatrone 1b usw. Die Substratpatrone 1a wird dann nach weiteren 30 Minuten aus dem Kreislauf entfernt. So erreicht man einen kontinuierlichen Verlauf der Titrationskurve.

Der Versuch wurde nach 256 Minuten bei einem Gesamtumsatz von ca. 90 % abgebrochen. Der Gesamtverbrauch an 5N NaOH beträgt 116,37 ml (90 %). Der Gesamtverbrauch an wäßriger Lösung beträgt 4,4 l. Wird die kontinuierlich abgezapfte wäßrige Lösung auf +6°C gekühlt, so kristallisiert noch zusätzlicher D-Ester aus, so daß das Konzentrationsverhältnis des Filtrats nun 83 : 17 (Säure : Ester) beträgt.

Die Rückstände in den Substratgefäßen werden getrocknet und analysiert. Die Ergebnisse sind in der folgenden Tabelle aufgeführt.

| Substrat-vorratsgefäß Nr.: | DL-Ester g/mol eingesetzt | $H_2O$ ml einges. | Rückstand D-Ester g/mol | % D-Ester als Feststoff isoliert |
|---|---|---|---|---|
| 1a | 44,26/0,2 | 500 | 15,45/0,07 | 70 % |
| 1b | 66,39/0,3 | 200 | 27,28/0,12 | 82 % |
| 1c | 88,52/0,4 | 50 | 33,02/0,15 | 75 % |
| 1d | 88,52/0,4 | 200 | 32,64/0,15 | 74 % |
| Summe | 287,70/1,3 | 950 | 108,39/0,49 | |

500 ml der wäßrigen Lösung (Teil des 4,4 l-Gesamtvolumens) werden auf 150 ml eingeengt und 2 mal mit 150 ml Methylenchlorid extrahiert. Nach dem Einengen der Methylenchloridphase am Rotationsverdampfer erhält man weitere 3,3 g des D-Esters. Die verbleibende Wasserphase wird am Rotationsverdampfer eingeengt, es verbleiben 22,20 g eines viskosen Öls, welches mit 150 ml 5 N HCl 2 h unter Rückfluß gekocht wird. Nach dem Einengen erhält man 14,4 g L-Phenylalaninhydrochlorid
optischer Drehwert: $\alpha_D^{20}$ = -10,5°C (c = 1, in $H_2O$)
Reinheit (GC mit chiraler Säule): 99,4 % L-Form

5 g von L-Phenylalaninhydrochlorid werden in 45 ml Ethanol und 5 ml $H_2O$ gelöst und mit 33 % NaOH auf einen pH-Wert von 6,12 gestellt. Die ausgefallene Festsubstanz wird mit Wasser gewaschen. Man erhält 2,60 g L-Phenylalanin.
optischer Drehtwert: $\alpha_D^{20}$ = -33,8°C (c = 1 in $H_2O$)

Beispiel 2

Racematspaltung von D,L-N-Acetyl-phenylalanin-n-propylester.
Man verfährt gemäß Beispiel 1 mit folgenden Varianten:

-Verwendung von nur einem Substratvorratsgefäß;
Füllmenge: 74,79 g (0,5 mol) D,L-N-Acetyl-phenylalaninn-propylester.
-Die Apparatur wird mit 550 ml Wasser gefüllt.
-Die Biokatalysatorsäule ist mit 5 ml immobilisiertem $\alpha$-Chymotrypsin gefüllt.
-Die Umpumpgeschwindigkeit beträgt 3,3 l/h
-Der Zu-/Ablauf beträgt 2 ml/h.

Aufgrund der geringeren Löslichkeit des Propylesters (0,5 %ige Lösung bei pH 7,0, T = 30°C) kristallisiert der nicht umgesetzte D-Ester rasch aus. Die relative Konzentration des Esters beträgt nach 4-5 h Reaktionszeit 24 %. In diesem Bereich kann kontinuierlich gezapft werden.

Die Reaktion wurde nach 15 Stunden abgebrochen. Zur Aufarbeitung verfährt man weiter gemäß Beispiel 1 und erhält 4,05 g L-Phenylalaninhydrochlorid mit einer optischen Reinheit (GC mit chiraler Säule) von 99,3 %.

Beispiel 3

Monoesterspaltung von L-N-Chloracetyl-glutaminsäurediethylester

10 g L-N-Chloracetyl-glutaminsäurediethylester werden in 300 ml Aceton gelöst und mit Kieselgelgranulat (Durchmesser 2,3 mm) (Shell S 970, G 2,3) versetzt. Man engt langsam am Rotationsverdampfer ein und behandelt die belegten Kugeln anschließend noch 30 Minuten im Hochvakuum. Sie werden dann in die Substratvorratsbehälter gegeben. Man füllt die in Beispiel 1 beschriebene Vorrichtung mit 400 ml Wasser und pumpt 10 min., um eine mit Substrat gesättigte wäßrige Lösunsg zu erhalten. Danach wird die Enzymsäule in den Kreislauf eingeschaltet. Man verfährt weiter gemäß Beispiel 1.

Nach 85 Minuten wurde die Reaktion abgebrochen. Nach den Ergebnissen der HPLC-Analyse erfolgt die Spaltung zu L-N-Chloracetylglutaminsäure-$\gamma$-monoethylester quantitativ.

Beispiel 4

Racematspaltung von D,L-N-Acetyl-phenyl-glycinethyl-ester

DL-N-Acetyl-phenylglycinethylester wird gemäß C.S.Marvel, J. Am. Chem. Soc. 42, 2265 (1920) hergestellt.

Zur Racematspaltung verfährt man gemäß Beispiel 1 füllt jedoch nur eine Säule mit 44,26 (0,1 mol) Substrat.

- Umpumpgeschwindigkeit: 4,5 l/h
- Zutropfgeschwindigkeit: 23,5 ml/h
- Spaltgeschwindigkeit: 4,5 mmol/h
- Raum/Zeit-Ausbeute: $\frac{34\ \text{g Phenylglycin}}{\text{kg} \cdot \text{h}}$

Die Reaktion wurde abgebrochen nachdem 89 % des Substrats umgesetzt waren.

Zur Aufarbeitung wird die noch im Reaktor befindliche Flüssigkeit mit dem abgepumpten Produkt vereinigt, auf 2/3 des Volumens eingeengt und auf 6°C abgekühlt. Anschließend wird der Feststoff abgesaugt. Man erhält 21,95 g D-Ester [$\alpha_D$ = 142,4°, c = 1 in $CH_3OH$].

Die verbleibende Wasserphase wird mit halbkonzentrierter HCl auf pH 2 eingestellt und der Feststoff abgesaugt. Man erhält 15,95 g L-N-Acetyl-phenylglycin [$\alpha_D$ = + 168,7°, c= 1 in $CH_3OH$].

## Ansprüche

1. Verfahren zur kontinuierlichen enzymatischen Umsetzung von in Wasser schwerlöslichen Substraten, dadurch gekennzeichnet, daß mittels einer wäßrigen Lösung kontinuierlich das in einem Behälter befindliche Substrat herausgelöst wird und über den in einem nachgeschalteten Behälter befindlichen Biokatalysator geführt wird.

2. Verfahren nach Anspruch 1, dadurch gekennzeichnet, daß als Substrat eine optische aktive Verbindung oder deren Racemat eingesetzt wird.

3. Verfahren nach Anspruch 2, dadurch gekennzeichnet,daß als Substrat ein aromatischer D,L-Aminosäureester oder ein L-α-Aminosäurediester bzw. deren Acylderivate eingesetzt werden.

4. Verfahren nach Anspruch 3, dadurch gekennzeichnet, daß als aromatischer D,L-Aminosäureester Tryptophan, Phenylalanin, Homophenylalanin, N-Acetyl-phenyl-glycinethylester, Tyrosin oder 3,4-Dihydroxyphenylalanin eingesetzt werden.

5. Verfahren nach Anspruch 3, dadurch gekennzeichnet, daß als α-Aminosäurediester L-Asparaginsäurediester oder L-Glutaminsäurediester oder deren Racemate eingesetzt werden.

6. Verfahren nach einem oder mehreren der Ansprüche 1 bis 5, dadurch gekennzeichnet, daß als Biokatalysator eine Esterase eingesetzt wird.

7. Verfahren nach Anspruch 6, dadurch gekennzeichnet, daß als Biokatalysator eine Serinprotease eingesetzt wird.

8. Verfahren nach Anspruch 7, dadurch gekennzeichnet, daß als Biokatalysator α-Chymotrypsin eingesetzt wird.

9. Vorrichtung zur Durchführung des Verfahrens nach Anspruch 1, gekennzeichnet durch

a) ein oder mehrere nebeneinandergeschaltete mit einem Kühl/Heizmantel versehene Gefäße (1a, b usw.) aus inertem Material, die das Substrat enthalten, mit einem Sieb (2a, b) im unteren Teil und

b) dem a) nachgeschalteten mit einem Kühl/Heizmantel versehenen Gefäß (3) aus inertem Material, das den Biokatalysator enthält, mit einem Sieb (4) im unteren Teil und

c) dem a) und b) nachgeschalteten mit einem Kühl/Heizmantel umgebenen Titrationsgefäß (5) aus inertem Material, das mit einem Rührer (6), einer Titrationsvorrichtung (7) und über eine Pumpe (8) mit einem Ablauf (9) sowie über eine weitere Pumpe (10) mit einem Zulauf (11) versehen ist,

wobei eine Umwälzpumpe (12) die wäßrige Lösung im Kreislauf vom Titrationsgefäß über

d) eine inerte Schlauchleitung (13) sowie über einen Dreiwegehahn (14a) und den Zulauf (15a, b usw.) in das Substratgefäß, von dort über

e) den Ablauf (16a, b usw.) und einen Dreiwegehahn (14b) durch die inerte Schlauchleitung (17) in das Biokatalysatorgefäß und weiter über

f) die inerte Schlauchleitung (18) in das Titrationsgefäß

pumpt.

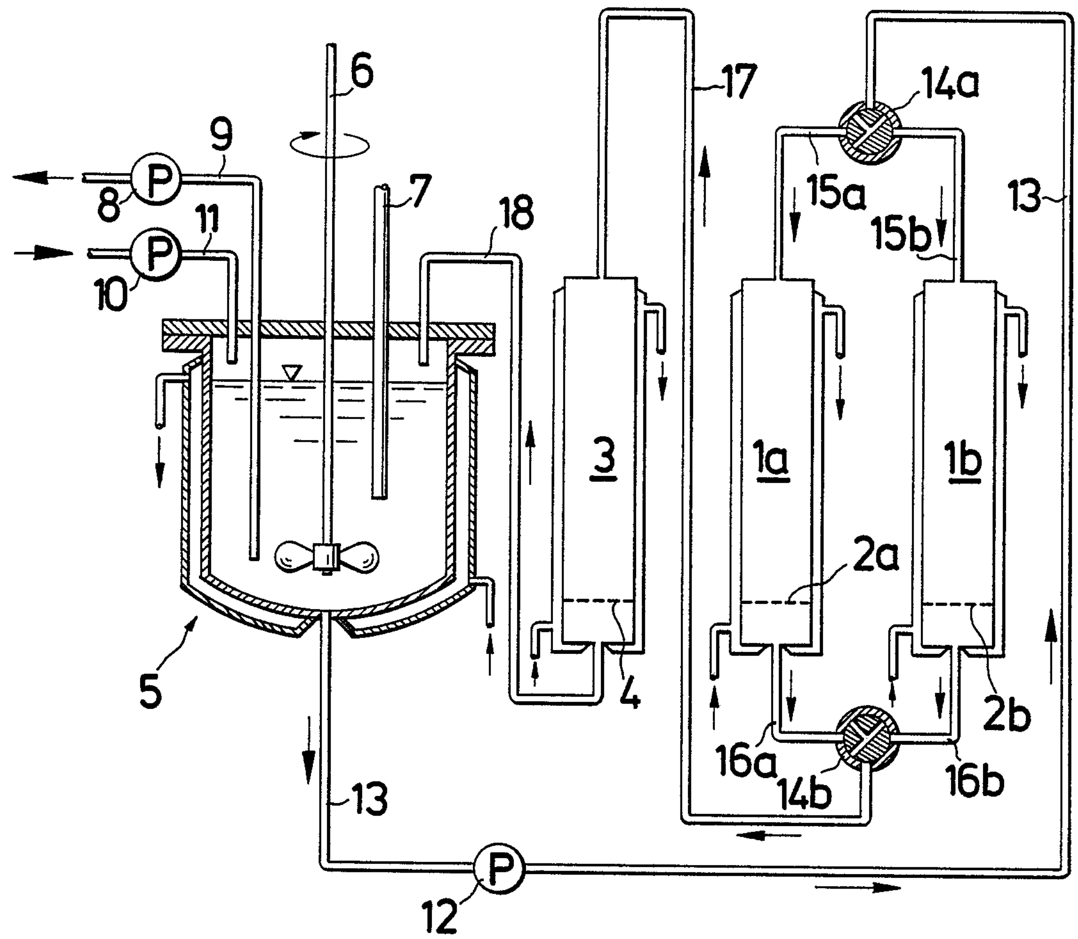
6
9
7
18
17
14a
8
11
15a
13
15b
10
3
1a
1b
2a
5
4
2b
16a
14b
16b
13
12